# EUROPEAN PATENT SPECIFICATION

(11) **EP 4 146 070 B1**
(45) Date of publication and mention of the grant of the patent: **11.11.2026**
(21) Application number: 21822457.4
(22) Date of filing: 10.06.2021
(51) Int. Cl.: A61B 5/107, A61B 90/00

(54) **SURGICAL SIZER SOUND**
CHIRURGISCHE GRÖSSENMESSSONDE
GABARIT D'ESSAYAGE CHIRURGICAL

(30) Priority: 11.06.2020 US 202063037911 P
(43) Date of publication of application: 15.03.2023
(73) Proprietor: LSI Solutions, Inc., Victor, NY 14564 (US); Sauer, Jude, S., Pittsford, NY 14534 (US)
(72) Inventor: SAUER, Jude, S., Pittsford, NY 14534 (US)
(74) Representative: Flügel Preissner Schober Seidel
(86) International application number: PCT/US2021/036798
(87) International publication number: WO 2021/252754

(56) References cited:
- WO-A1-2020/092422
- WO-A1-96/39942
- US-A- 5 682 906
- US-A- 5 713 951
- US-A- 6 019 739
- US-A1- 2002 111 679
- US-A1- 2010 249 661
- US-A1- 2011 098 602
- US-A1- 2015 012 086
- US-A1- 2015 012 086

## Description

### RELATED APPLICATIONS

This application claims priority to U.S. Provisional Patent Application No. 63/037,911, which was filed on June 11, 2020 and entitled "SURGICAL SIZER SOUND".

### FIELD

The claimed invention relates to surgical sizing devices, and more specifically to a surgical sizer sound.

### BACKGROUND

Modern advances in cardiac surgery have made it possible to replace heart valves using minimally invasive surgical techniques. As minimally invasive techniques have improved, surgeons are able to operate on patients through smaller and smaller access holes, resulting in less perioperative pain and shorter recovery times. A main focus of innovations in minimally invasive cardiac surgery has been on the tools which pass into the patient, through the small access holes, and ensuring these surgical tools and devices can be effectively used within the dimensions of a smaller access hole. By focusing on improvements to these elements of the surgical procedures, patients are able to be on cardio-pulmonary bypass machines for shorter times, thereby improving patient outcomes. Resultant efficiency improvements while working within the patient further help to reduce stress and fatigue on surgeons.

If one or more of a person's heart valves developing problems which adversely affect their function can, consequently, it can negatively impact the person's health. Generally, problems with heart valves can be organized into two categories: regurgitation and/or stenosis. Regurgitation occurs if a heart valve does not seal tightly, thereby allowing blood to flow back into a chamber rather than advancing through and out of the heart. This can cause the heart to work harder to remain an effective pump. Regurgitation is frequently observed when the mitral valve prolapses (extends back) into the left atrium during a ventricular contraction. Stenosis, by contrast, is when a heart valve does not fully patent due to stiff or fused leaflets, blood flow tract hours of which can be spent on a CPB machine. While the patient is on CPB, the surgeon must gain access to the heart valve, remove the pathologic valve tissue as necessary, and install a replacement valve at the location of the original valve. The valve installation process can be very time consuming, especially when surgeons are operating through small access sites when employing less-invasive techniques to reduce surgical trauma. Therefore, there is a need for devices and methods which enable surgeons to operate more efficiently during surgery to replace pathologic anatomical structures, such as, but not limited to, replacement heart valves. Such devices and methods can reduce the amount of time patients need to be attached to a CPB machine, thereby reducing the likelihood of CPB-related side effects. Devices and methods enabling surgeons to operate more effectively while utilizing smaller access sites to a surgical site may further result in benefits to the patient in terms of decreased pain, improved cosmetics, reduced hospital stays, and accelerated recovery.

WO 96/39942 A1 discloses a replacement valve sizing disk. The valve sizing disk includes a disk body. Disk body has a shape corresponding to that of the prosthetic valve to be used for replacing the native valve, and is usually circular. A handle coupling is mounted to the disk body. Handle coupling comprises a slot configured to receive holder coupling of delivery handle. The sizing disk when attached to coupling means of delivery handle may be introduced through an intercostal space without retraction or removal of ribs.

US 2015/012086 A1 and US 6 019 739 A disclose a handle that can be screwed into a sizer.

US 5 682 906 A discloses a device that allows for a method of closed-chest surgical intervention within an internal cavity of a patient's heart or great vessel.

The invention is set out in the appended set of claims.

### BRIEF DESCRIPTION OF THE DRAWINGS

FIG. 1 is a top front left perspective view of a surgical sizer sound.
FIG. 2. is a bottom front left perspective view of the surgical sizer sound of FIG. 1.
FIGS. 3A and 3B are a top view and a perspective view, respectively, of a sizer of the surgical sizer sound of FIG. 1.
FIGS. 4A and 4B are left side and perspective views, respectively, of a sizer coupler of the surgical sizer sound of FIG. 1.
FIG. 5A is a perspective view of the sizer being loaded onto the sizer coupler.
FIG. 5B is a perspective view of the sizer attached to the sizer coupler, illustrating the range of motion of the sizer coupler of the surgical sizer sound of FIG. 1.
FIGS. 6A and 6B are partial cross-sectional views illustrating various loading positions of the sizer onto the sizer coupler of the of the surgical sizer sound of FIG. 1.
FIGS. 7A-7E are side, partial cross-sectional views of the surgical sizer sound of FIG. 1 during a surgical procedure introducing the surgical sizer sound between two ribs.
FIG. 8 is a bottom front left perspective view of an alternate embodiment of a surgical sizer sound.

It will be appreciated that for purposes of clarity and where deemed appropriate, reference numerals have been repeated in the figures to indicate corresponding features, and that the various elements in the drawings have not necessarily been drawn to scale in order to better show the features.

### DETAILED DESCRIPTION

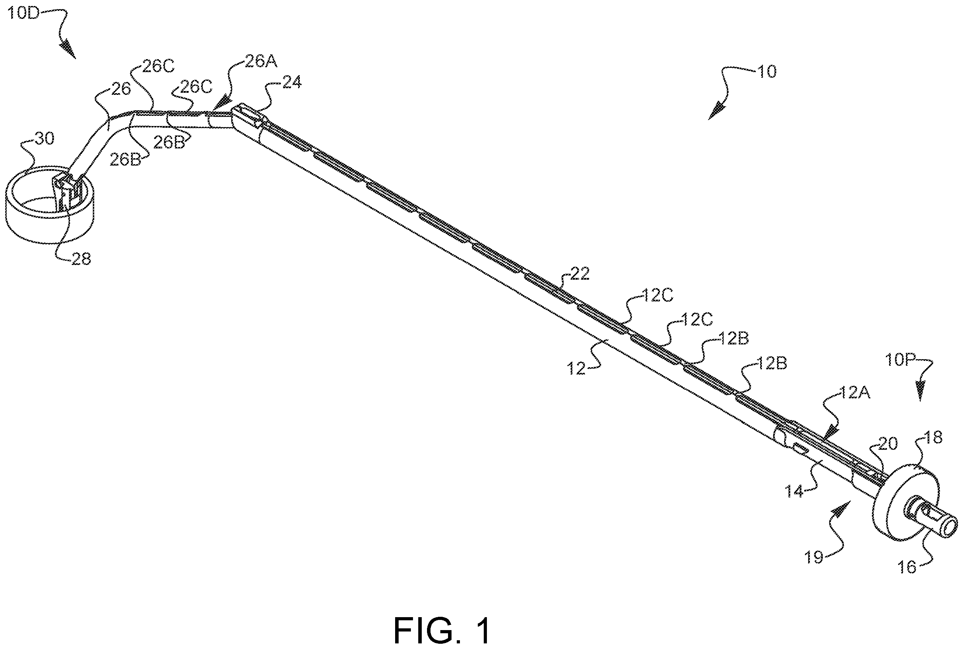

FIG. 1 is a top front left perspective view of a surgical sizer sound. An articulating, angled sizer sound as illustrated may be used in a minimally invasive coronary valve replacement surgical procedure. The sound 10, as described, is may be provided as a sterile, cleanable, and reusable product that is used to probe and couple to various coronary structures. The sound 10 is centrally comprised of a substantially cylindrical guide shaft 12, which towards a distal end 10D is coupled to an angled shaft mount 24 which is further coupled to an angled shaft 26. A sizer coupler 28 is pivotably coupled to the angled shaft 26, and a sizer 30 is removably coupled to the sizer coupler 28. A handle 14 is coupled to the guide shaft 12, and a rotating knob 18 and an end cap 16 are disposed at a proximal end 10P of the sound 10. A clockwise rotation 19 of the rotational knob 18 of the sound 10 will cause a drive screw 20 and attached drive wire 22, to retract and induce a pulling force on the drive wire 22 housed within the sizer coupler 28 on the distal end 10D of the sound 10. This pulling will cause the sizer coupler 28, and therefore the attached sizer 30, to follow rotation direction 70, pivoting about a pivot point, and move perpendicular to the angled shaft 26. Conversely rotating the rotational knob 18 in the opposite direction will impart a pushing force on the drive screw 20 and also the drive wire 22, causing the sizer coupler 28 to move in opposite direction 68 to return to its original position relative to the angled shaft 26. The full range of motion in the illustrated embodiment is approximately 90 degrees, although alternate embodiments may have a range of motion more than 90 degrees or may pivot in more than one direction relative to the angled shaft 26. Alternatively, using a reverse threaded rotatable knob 18 could provide for opposite actuation between the perpendicular and aligned positions. Alternate embodiments having an actuatable shaft distal end may incorporate an alternate operating mechanism such as a lever, slider, or other means known to those skilled in the art. As described previously, the drive screw 20 is engaged with the rotational knob 18 to push or pull the drive wire 22 which engages with and directs the sizer coupler 28. The drive wire 22 emanating from the drive screw 20 is delivered through the guide shaft 12 via a wire channel 12A, where it is retained by retaining tabs 12B and allowed cleaning access via cleaning troughs 12C. Lastly, the wire also passes through the angled shaft 26 via a wire channel 26A, where it is retained by retaining tabs 26B and allowed cleaning access via cleaning troughs 26C. At the distal end of the wire channel 26A, the wire 22 connects to the sizer coupler 28. Alternate embodiments may have shafts that are actuatable by means other than a rotational knob and drive screw, and may incorporate other operational or actuatable shafts by means such as levers, sliders, or other mechanical means known to those skilled in the art.

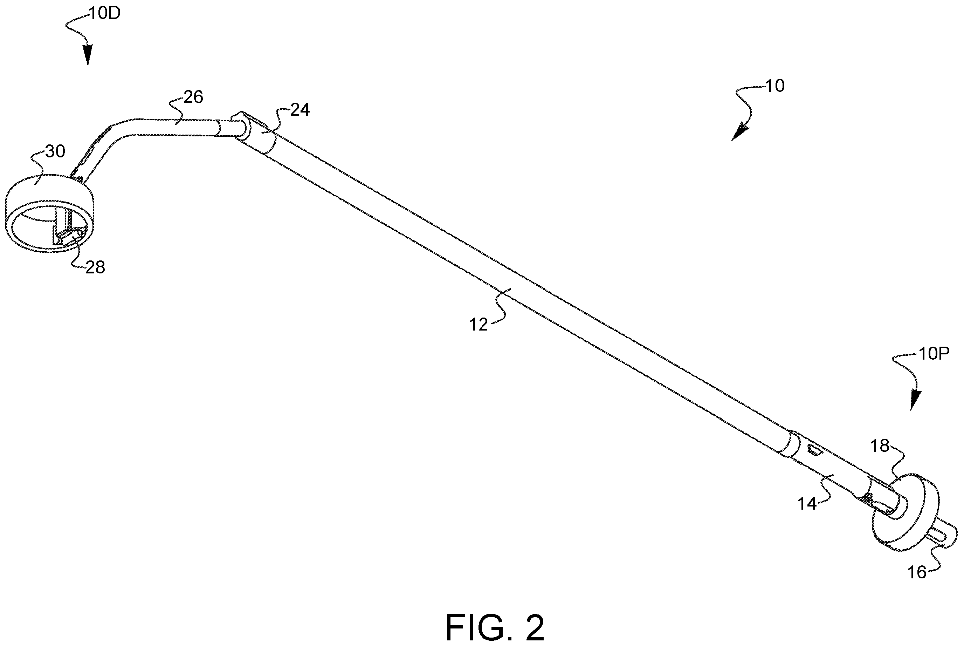

It should be noted that due to variations in the human anatomy, different sizes of devices or individual components therein may be required from one surgical case to the next and the size of a heart and each annulus must be measured prior to selecting the appropriate size of a heart valve replacement. The sound 10 can be manufactured and offered with a variety of angled shafts 26 to accommodate an array of anatomical differences. Further, multiple dimensions relative to the sizer 30 may be provided depending on these anatomical variations from patient to patient. For example, a complement of six sizers having outer diameters of 19, 21, 23, 25, 27 and 29mm in a surgical procedure kit may be provided. Alternatively, additional sizers in even increments such as 20mm, 22mm, or others may also be provided. Additional considerations may be addressed when a minimally invasive valve replacement valve replacement via microthoracotomy procedure is performed, such as angle of approach, dimensions of the sizer loaded onto the distal end, adjustability, and maneuverability. Alternate embodiments may include further articulation or angling points on the angled shaft 26 or incorporate a flexible or bendable angled shaft for setting and resetting a specific angle or profile on the angled shaft 26 relative to the guide shaft 12. The sound and related components described are manufactured from a non-corrosive, biocompatible, rigid, and sterilizable material, such as stainless steel. The sizer 30 may alternately be fabricated as a non-reusable component and from a biocompatible yet somewhat compressible or compliant material. Similar sounds used in minimally invasive surgical procedures and the assembly and associated methods thereof have been disclosed in U.S. Pat. No. 8,603,105 and in U.S. Pat. No. 10,092,323. FIG. 2. is a bottom front left perspective view of the surgical sizer sound of FIG. 1.

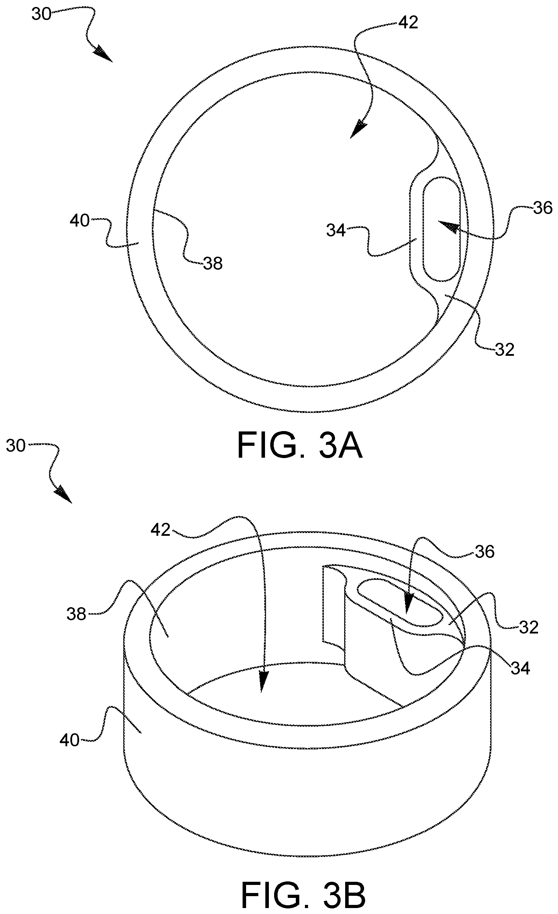

FIGS. 3A and 3B are a top view and a perspective view, respectively, of a sizer of the surgical sizer sound of FIG. 1. FIG. 3A is a top view of a sizer for use in the surgical sizer sound 10 of FIG. 1. The sizer 30 is comprised of a single component as a substantially hollow cylinder. The sizer 30 defines a mounting feature 32 on an inner circumference or inner wall 38, the mounting feature 32 having a sizer catch 34 and further defining a sizer mount opening 36. The sizer mount opening 36 is configured such that it corresponds to the dimensions and orientation of several features on the sizer coupler 28, which will be described in more detail later. The sizer 30 also defines an outer wall 40 located on the outer circumference of the sizer 30. The outer diameter defined by the outer wall 40 of the sizer 30 determine the size of the sizer 30 used in conjunction with the sizer sound 10 as releasably attached to the sizer coupler 28 during a minimally invasive cardiac valve replacement procedure. While the sizer 30 illustrated in FIGS. 3A and 3B is comprised of a single component, alternative embodiments may include a sizer with a mounting feature 32 that is fixedly attached or otherwise coupled to the inner circumference of the sizer 30. The mounting feature 32 is configured to engage and allow the sizer 30 to be releasably held the onto the sizer coupler 28 in a single, intended orientation. FIG. 3B is a perspective view of the sizer of FIG. 3A.

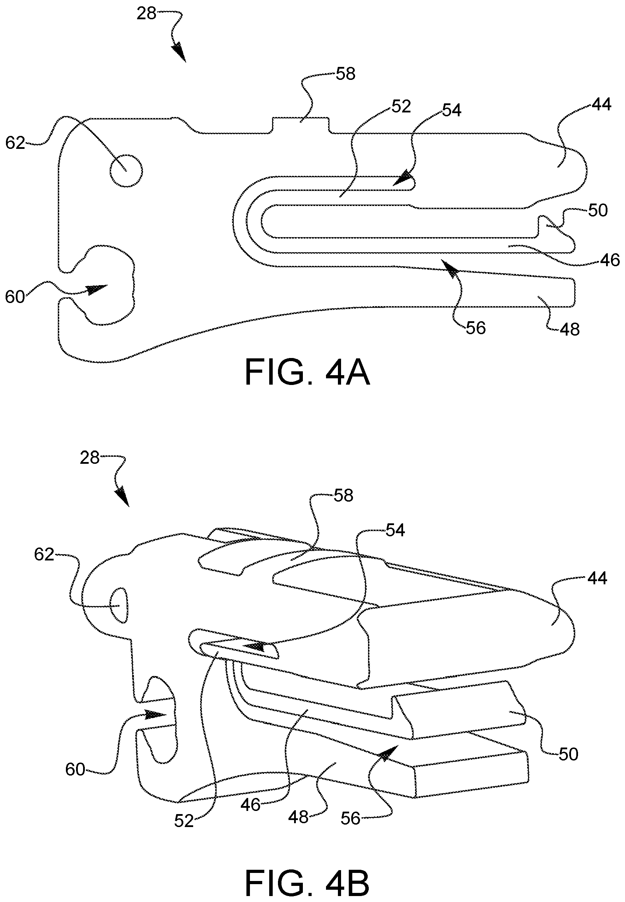

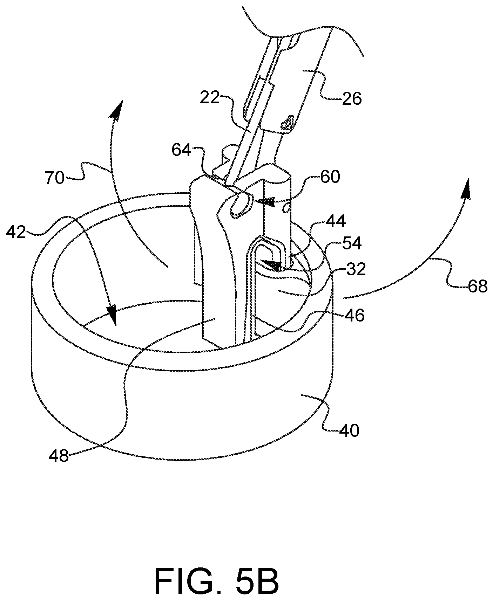

FIGS. 4A and 4B are left side and perspective views, respectively, of a sizer coupler of the surgical sizer sound of FIG. 1. The sizer coupler 28 a pivot point 62 and an actuator coupler 60 on one end. The actuator coupler 60 is configured to receive the drive wire 22 and is pivotably actuated about its pivot point 62 when the rotational knob 18 is actuated or rotated as described previously. Further details will be described in regard to FIG. 5B. The sizer coupler 28 also defines a guide tip 44, which is shaped and configured to interface with, receive, and release the sizer 30 during loading, use and unloading of the sizer sound 10 during a minimally invasive microthoracotomy involving a valve replacement procedure. The end of the guide tip 44 is rounded in order to facilitate the insertion of the guide tip 44 into a corresponding feature on the sizer 30. The guide tip 44 further defines a stop 58 which is a substantially rectangular-shaped protrusion configured to prevent a sizer from being inserted too far onto the guide tip 44. On the opposite side of the sizer coupler 28, the sizer coupler 28 further defines a limit 48. The limit 48 is coupled to the guide tip 44 by an internally curvilinear bridge. The guide tip 44 also includes a retainer 46 coupled to the guide tip 44 by a biasing element 52, the retainer 46 terminating in a latch 50 on its distal end. The guide tip 44, retainer 46, and limit 48 are substantially parallel and configured to receive a sizer 30 in a single orientation while preventing or reducing the possibility of loading a sizer 30 onto the sizer coupler 28 in an improper orientation. Between the guide tip 44 and the element 52 portion of the retainer 46, the sizer coupler 28 defines a first inner clearance gap. Between the limit 48 and the retainer 46, the sizer coupler 28 defines a second inner clearance gap 56. The biasing element 52 and retainer 46 have some flexibility such that the retainer 46 may flex towards the limit 48 to allow for the insertion of a sizer 30 onto the sizer coupler 28 between the guide tip 44 and retainer 46. The retainer 46 may also flex towards the limit 48 but cannot flex beyond the location of the limit 48, thus limiting the travel of the retainer 46 in a direction towards the limit 48 and away from the guide tip 44. The presence of the limit 48 on the sizer coupler 28 prevents the retainer 46 from becoming excessively flexed and potentially maintaining a flexed position after repeated use. While the sizer coupler shown is a singular piece, alternate embodiments may be comprised of separate components or multiple pieces assembled together.

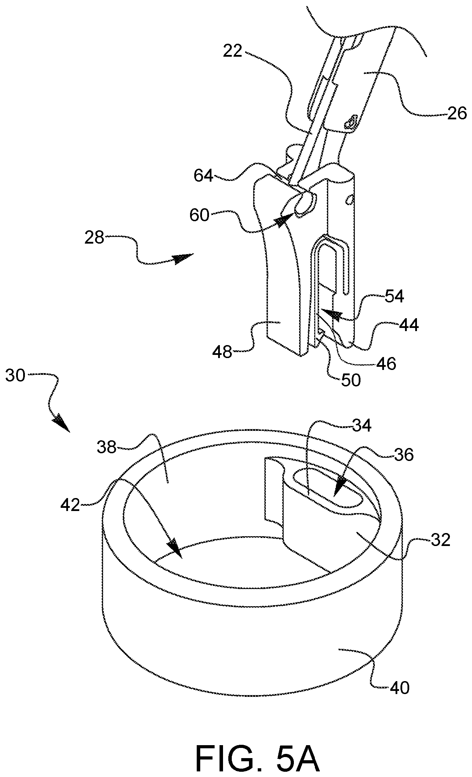

FIG. 5A is a perspective view of the sizer being loaded onto the sizer coupler. FIG. 5A illustrates the appropriate positioning of the sizer 30 while being loaded onto the sizer coupler 28 of the sizer sound 10. This view of the distal end 10D of the sizer sound 10 illustrates the detail of the anchor nut 22 and terminating drive wire coupler 64 as seated into the actuator coupler 60 of the sizer coupler 28. The complementary shape and orientation of the guide tip 44, retainer 46, and limit 48 on the sizer coupler 28 are oriented such that the guide tip 44 enters the sizer mount opening 36 on the sizer 30, the ramp-like shape of the latch 50 on the retainer 46 rides up and over the internal side of the sizer catch 34. The limit 48 prevents the retainer 46 from excessively flexing until the latch 50 clicks into place onto the edge of the sizer catch 34. This temporarily locks the sizer 30 onto the end of the sizer coupler 28 until such time that the latch 50 can be intentionally defeated to release the latch 50 and retainer 46 from the sizer catch 34 and allow removal of the sizer 30 from the sizer coupler 28. This fully loaded and locked configuration is illustrated in FIG. 5B. The described latch 50 defeat operation may be accomplished with the use of surgical tools such as graspers or other tools suitable for extension into a surgical port along with the sizer sound 10. FIG. 5B is a perspective view of the sizer attached to the sizer coupler, illustrating the range of motion of the sizer coupler of the surgical sizer sound of FIG. 1. As previously described, when the rotational knob 18 is rotated in a clockwise direction 19, the drive screw 20 and attached drive wire 22, are retracted and induce a pulling force on the drive wire 22 held within the actuator coupler 60 on the sizer coupler 28 on the distal end 10D of the sound 10. This pulling will cause the sizer coupler 28, and therefore the attached sizer 30, to follow rotation direction 70, pivoting about a pivot point 62, and move perpendicular to the angled shaft 26. Conversely rotating the rotational knob 18 in the opposite direction will impart a pushing force on the drive screw 20 and also the drive wire 22, causing the sizer coupler 28 to move in opposite direction 68 to return to its original position relative to the angled shaft 26 as illustrated in FIG. 5B.

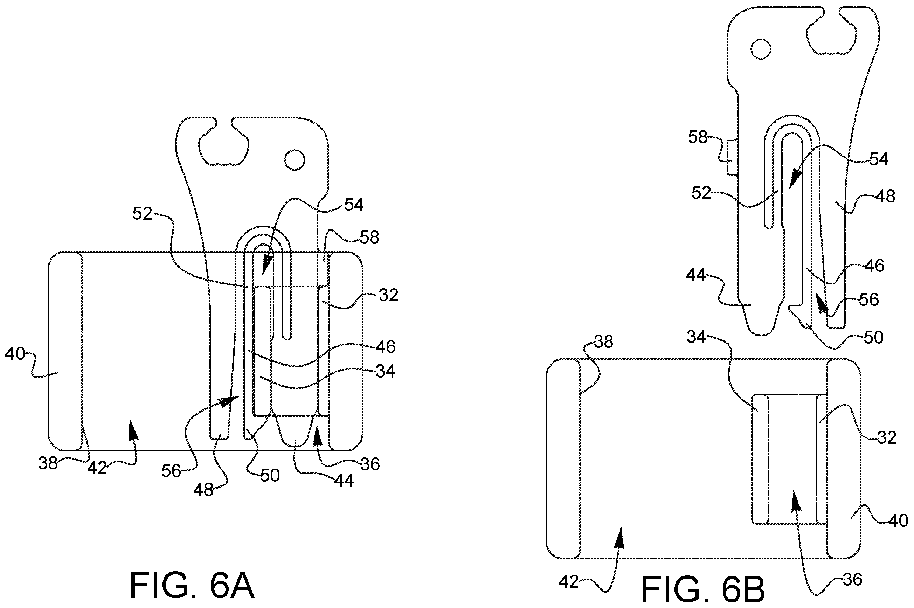

FIGS. 6A and 6B are partial cross-sectional views illustrating various loading positions of the sizer onto the sizer coupler of the of the surgical sizer sound of FIG. 1. FIG. 6A illustrates an appropriate loading orientation of the sizer 30 onto the sizer coupler 28. Several features of the sizer sound 10 are removed in FIGS. 6A and 6B for the purposes of clarity. The shape of the guide tip 44 is configured to fit into the sizer mount opening 36 of the sizer 30. The retainer 46 and limit 48 are configured to encourage insertion of the guide tip 44 of the sizer coupler 28 into the sizer mount opening 36 of the mounting feature 32 of the sizer 30 in only the orientation shown in FIG. 6A. The limit 48 prevents the retainer 46 from excessively flexing on the biasing element 52 attached to the guide tip 44 until the latch 50 of the retainer 46 clicks into place onto the edge of the sizer catch 34 once the guide tip 44 is fully inserted into the sizer mount opening 36 of the sizer 30. The retainer 46 may flex and travel through the second inner clearance gap 56 until the retainer 46 contacts the limit 48. Once the latch 50 has clicked into place on the edge of the sizer catch 34, the sizer catch 34 is seated within the first inner clearance gap 54 between the retainer 46 and the guide tip 44. The sizer coupler 28 and guide tip 44 are prevented from being further inserted into the sizer mount opening 36 by interference between the mounting feature 32 and the stop 58. Later removal of the sizer 30 from the sizer coupler 28 can be accomplished by mechanically defeating the latch 50 by pushing the latch 50 and retainer 46 through the second inner clearance gap 56 towards the limit 48 to clear the latch 50 from its holding position on the sizer catch 34 of the mounting feature 32 on the sizer 30. FIG. 6B shows an opposite orientation and positioning of the sizer coupler 28 being inserted into the sizer 30. While the guide tip 44 would fit into the sizer mount opening 36 of the mounting feature 32 on the sizer 30, the locations, position and configuration of the first inner clearance gap 54, retainer 46, second inner clearance gap 56, and limit 48 would impede or prohibit the insertion of the guide tip 44 on the sizer coupler 28 into the sizer mount opening 36 of the mounting feature 32 of the sizer 30. While the sizer 30 could be loaded from either side, it can only be loaded onto the sizer coupler 28 in one orientation, with the sizer catch placed within the first inner clearance gap 54 with the latch 50 and retainer 46 on the inner portion of the sizer catch 34 of the mounting feature 32 as shown in FIG. 5A. This described configuration and interlocking and complementary features on the sizer coupler 28 and sizer 30 are advantageous in preventing or reducing the possibility of misplacing or loading a sizer 30 onto the sizer sound 10 in a manner that would prevent the instrument from operating correctly or having a sizer become inadvertently dislodged.

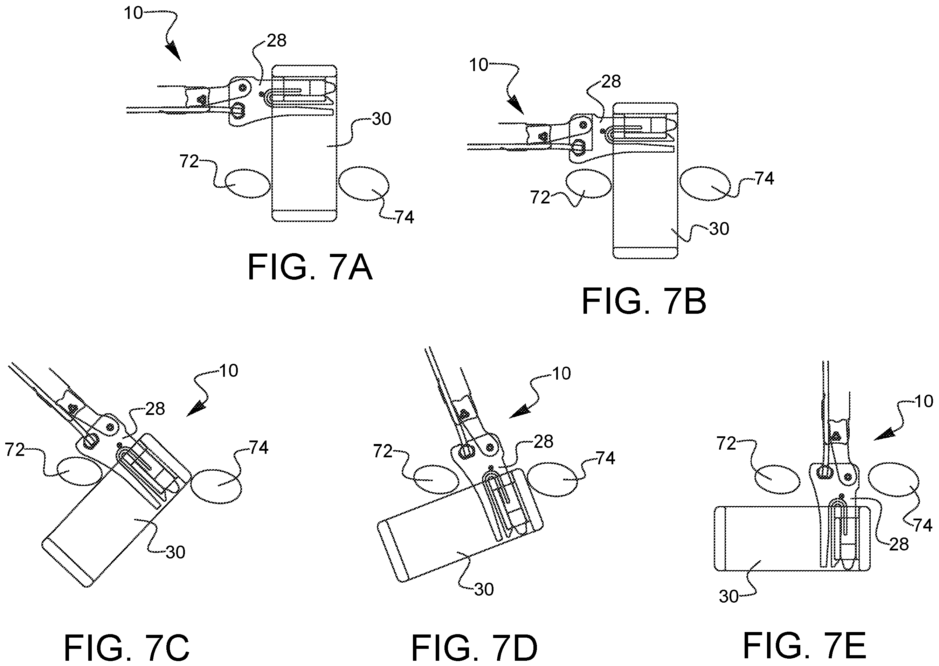

FIGS. 7A-7E are side, partial cross-sectional views of the surgical sizer sound of FIG. 1 during a surgical procedure introducing the surgical sizer sound between two ribs. An over all microthoracotomy may consist of several general steps, only some of which, relevant to a sizer sound device, are described herein. Once access for a microthoracotomy is established, typically via at least one small incision wherein a scope port or other access port is established, cardio-pulmonary bypass is also established. Aortic cross clamping, aortotomy, and aortic valve debridement and optional stabilization are subsequently conducted. Annular sizing is commonly a next step in a minimally invasive valve replacement procedure. The surgical sizer sound is introduced intercostally while actuating the surgical sizer sound to minimize interference with one or more ribs. The surgical sizer sound is then advanced such that the sound attachment is inserted into the appropriate annulus which is the target site of the valve replacement. The sizer and successively larger sizers are inserted into the annulus until an adequate and complete fit of the largest possible sizer is determined. This size is used as the optimal valve size choice. A replica attachment may also be attached to the sizer coupler where applicable to verify the correct valve size. FIG. 7A is a side view partial cross-section of an internal view of an intercostal space surrounded by two ribs 72, 74, which is a typical insertion point for a sizer sound 10 such as the one described herein. Usually a minimally invasive surgical procedure involving a microthoracotomy utilizes either the second or third intercostal space between the ribs. The orientation of the sizer coupler 28 relative to the angled shaft 26 is shown here as neutral, although any angle of the sizer coupler 28 relative to the angled shaft 26 could be utilized based on present surgical conditions and preference of the surgeon. The sizer 30 is loaded onto the sizer coupler 28 as previously described herein. The sizer is placed between rib 72 and rib 74 and guided therethrough by the sizer sound 10. In some instances, although not shown here, rib retractors or other means may be used to further separate the ribs 72, 74 to gain increased intercostal space. FIG. 7B illustrates the sizer 30 being advanced further into the intercostal space between the ribs 72, 74, guided by the sizer sound 10. As the sizer sound 10 and more specifically the sizer coupler 28 interferes with rib 72, it is necessary to further angulate the distal end 10D of the sizer sound 10 in a manner that allows the sizer 30 to be further advanced atraumatically through the intercostal space. Further angulation and positioning of the sizer 30 may be necessary, as shown in FIG. 7C, which may be achieved via rotation or angulation of the entire sizer sound 10, as well as by articulating the sizer coupler 28 portion of the device relative to the angled shaft 26. The sizer 30 is in a position to be further guided around the ribs 72, 74 on a continuing atraumatic path towards the heart. FIG. 7D illustrates the sizer 30 having passed mostly through the intercostal space between the ribs 72, 74 and finally passed entirely through the intercostal space, as illustrated in FIG. 7E. Subsequently, the sizer 30 may be advanced, angled, and articulated as necessary to place the sizer 30 within the aortic annulus, in the case of an aortic valve replacement, for proper sizing and fit of the annulus for the purpose of utilizing the most appropriately sized replacement valve for the individual anatomical considerations of the patient.

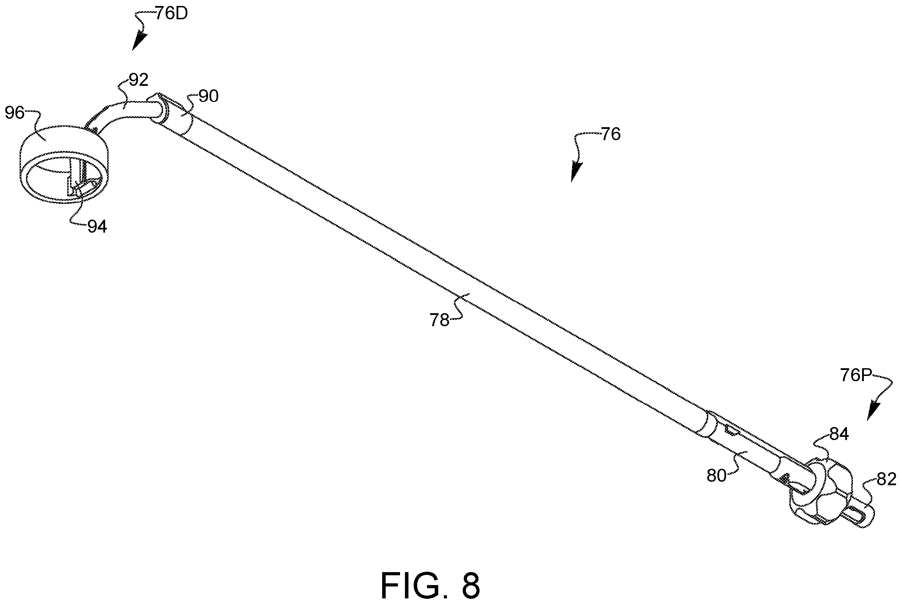

FIG. 8 is a bottom front left perspective view of an alternate embodiment of a surgical sizer sound. A sizer sound 76 having a guide shaft 78 with an angled shat mount 90 disposed towards the distal end 76D of the sizer sound 76, the angled shaft mount 90 coupled to an angled shaft 92. Similar to previous embodiments, the sizer sound 76 includes a sizer coupler 94 pivotably coupled to the angled shaft 92. A sizer 96 is releasably coupled to the sizer coupler 94 on the distal end 76D. A handle 80 is coupled to the guide shaft 78, and a rotating knob 84 and an end cap 82 are also disposed at a proximal end 76P of the sound 76. The knob 84 shown in FIG. 8 has a more ergonomic design for improved handling and grip. The operating principles and internal components of this embodiment of a sizer sound 76 are similar to that of the embodiment illustrated in FIG. 1 and described previously herein.

## Claims

1. A surgical sizer sound (10, 76) for probing and coupling to various coronary structures, comprising:
an actuatable shaft (12, 78) comprising an operating mechanism (18) operably coupled to a drive wire (22), wherein the operating mechanism (18) is configured to induce a pulling force or a pushing force on the drive wire (22);
an angled shaft (26, 92) coupled to the actuatable shaft (12, 78);
a sizer coupler (28, 94) pivotably coupled to the angled shaft (26, 92), comprising:
a pivot point (62),
a guide tip (44),
a limit (48),
an actuator coupler (60) connected to the drive wire (22),
a retainer (46) coupled to the guide tip (44) comprising a latch (50) on a distal end, and
a biasing element (52) coupled to the guide tip (44) and the retainer (46); and
a sizer (30, 96), removably coupled to the sizer coupler (28, 94);
wherein (a) when the operating mechanism (18) induces the pulling force on the drive wire (22) to retract, the sizer coupler (28) pivots about the pivot point (62) in a first rotation direction (70); and
wherein (b) when the operating mechanism (18) induces a pushing force on the drive wire (22), the sizer coupler (28) pivots about the pivot point (62) in a second rotation direction (68) opposite the first rotation direction (70).

2. The surgical sizer sound (10, 76) of claim 1, wherein the sizer coupler (28, 94) further comprises a stop (58).

3. The surgical sizer sound (10, 76) of claim 1 or 2, wherein the sizer coupler (28, 94) further comprises a first inner clearance gap (54).

4. The surgical sizer sound (10, 76) of claim 3, wherein the first inner clearance gap (54) is between the retainer (46) and the guide tip (44).

5. The surgical sizer sound (10, 76) of any one of claims 1 to 4, wherein the sizer coupler (28, 94) further comprises a second inner clearance gap (56).

6. The surgical sizer sound (10, 76) of claim 5, wherein the second inner clearance gap (56) is between the retainer (46) and the limit (48).

7. The surgical sizer sound (10, 76) of any one of claims 1 to 6, wherein the guide tip (44) and the limit (48) are coupled by an internally curvilinear bridge.

8. The surgical sizer sound (10, 76) of any one of claims 1 to 7, wherein the sizer coupler (28, 94) is formed of a singular piece.

9. The surgical sizer sound (10, 76) of any one of claims 1 to 8, wherein the sizer (30, 96) further comprises a hollow cylinder.

10. The surgical sizer sound (10, 76) of any one of claims 1 to 9, wherein the sizer (30, 96) further comprises a mounting feature (32) coupled to an inner circumference (38) of the sizer (30, 96).

11. The surgical sizer sound (10, 76) of claim 10, wherein the mounting feature (32) further comprises an opening (36).

12. The surgical sizer sound (10, 76) of any one of claims 1 to 11, wherein the sizer (30, 96) further comprises a sizer catch (34).

13. The surgical sizer sound (10, 76) of any one of claims 1 to 12, wherein the operating mechanism is a rotatable knob (18).

14. The surgical sizer sound (10, 76) of any one of claims 1 to 13, wherein the angled shaft (26, 92) is articulable.

## Patentansprüche

1. Chirurgische Kalibriersonde (10, 76) zum Sondieren und Koppeln an verschiedene koronare Strukturen, umfassend:
einen betätigbaren Schaft (12, 78), umfassend einen Betätigungsmechanismus (18), der mit einem Antriebsdraht (22) wirkverbunden ist, wobei der Betätigungsmechanismus (18) eingerichtet ist, eine Zugkraft oder eine Druckkraft auf den Antriebsdraht (22) auszuüben;
einen abgewinkelten Schaft (26, 92), der mit dem betätigbaren Schaft (12, 78) gekoppelt ist;
einen Kalibrierelement-Koppler (28, 94), der schwenkbar mit dem abgewinkelten Schaft (26, 92) gekoppelt ist, umfassend:
einen Schwenkpunkt (62),
eine Führungsspitze (44),
ein Begrenzungselement (48),
einen Betätigungskoppler (60), der mit dem Antriebsdraht (22) verbunden ist,
einen Halter (46), der mit der Führungsspitze (44) gekoppelt ist und eine Verriegelung (50) an einem distalen Ende umfasst, und
ein Vorspannelement (52), das mit der Führungsspitze (44) und dem Halter (46) gekoppelt ist; und
ein Kalibrierelement (30, 96), das lösbar mit dem Kalibrierelement-Koppler (28, 94) gekoppelt ist;
wobei (a), wenn der Betätigungsmechanismus (18) die Zugkraft auf den Antriebsdraht (22) ausübt, um diesen zurückzuziehen, der Kalibrierelement-Koppler (28) um den Schwenkpunkt (62) in einer ersten Drehrichtung (70) schwenkt; und
wobei (b), wenn der Betätigungsmechanismus (18) eine Druckkraft auf den Antriebsdraht (22) ausübt, der Kalibrierelement-Koppler (28) um den Schwenkpunkt (62) in einer zweiten Drehrichtung (68) entgegengesetzt zu der ersten Drehrichtung (70) schwenkt.

2. Chirurgische Kalibriersonde (10, 76) nach Anspruch 1, wobei der Kalibrierelement-Koppler (28, 94) ferner einen Anschlag (58) umfasst.

3. Chirurgische Kalibriersonde (10, 76) nach Anspruch 1 oder 2, wobei der Kalibrierelement-Koppler (28, 94) ferner einen ersten inneren Freiraum (54) umfasst.

4. Chirurgische Kalibriersonde (10, 76) nach Anspruch 3, wobei der erste innere Freiraum (54) zwischen dem Halter (46) und der Führungsspitze (44) liegt.

5. Chirurgische Kalibriersonde (10, 76) nach einem der Ansprüche 1 bis 4, wobei der Kalibrierelement-Koppler (28, 94) ferner einen zweiten inneren Freiraum (56) umfasst.

6. Chirurgische Kalibriersonde (10, 76) nach Anspruch 5, wobei der zweite innere Freiraum (56) zwischen dem Halter (46) und dem Begrenzungselement (48) liegt.

7. Chirurgische Kalibriersonde (10, 76) nach einem der Ansprüche 1 bis 6, wobei die Führungsspitze (44) und das Begrenzungselement (48) durch eine innen gekrümmte Brücke gekoppelt sind.

8. Chirurgische Kalibriersonde (10, 76) nach einem der Ansprüche 1 bis 7, wobei der Kalibrierelement-Koppler (28, 94) einstückig ausgebildet ist.

9. Chirurgische Kalibriersonde (10, 76) nach einem der Ansprüche 1 bis 8, wobei das Kalibrierelement (30, 96) ferner einen Hohlzylinder umfasst.

10. Chirurgische Kalibriersonde (10, 76) nach einem der Ansprüche 1 bis 9, wobei das Kalibrierelement (30, 96) ferner ein Befestigungselement (32) umfasst, das mit einem Innenumfang (38) des Kalibrierelements (30, 96) gekoppelt ist.

11. Chirurgische Kalibriersonde (10, 76) nach Anspruch 10, wobei das Befestigungselement (32) ferner eine Öffnung (36) umfasst.

12. Chirurgische Kalibriersonde (10, 76) nach einem der Ansprüche 1 bis 11, wobei das Kalibrierelement (30, 96) ferner eine Kalibrierelement-Raste (34) umfasst.

13. Chirurgische Kalibriersonde (10, 76) nach einem der Ansprüche 1 bis 12, wobei der Betätigungsmechanismus ein Drehknopf (18) ist.

14. Chirurgische Kalibriersonde (10, 76) nach einem der Ansprüche 1 bis 13, wobei der abgewinkelte Schaft (26, 92) gelenkig bewegbar ist.

## Revendications

1. Sonde de calibrage chirurgicale (10, 76) destinée à sonder et à être couplée à diverses structures coronaires, comprenant :
une tige actionnable (12, 78) comprenant un mécanisme d'actionnement (18) couplé fonctionnellement à un fil d'entraînement (22), le mécanisme d'actionnement (18) étant configuré pour exercer une force de traction ou une force de poussée sur le fil d'entraînement (22) ;
une tige coudée (26, 92) couplée à la tige actionnable (12, 78) ;
un coupleur de calibreur (28, 94) couplé de manière pivotante à la tige coudée (26, 92), comprenant :
un point de pivotement (62),
une pointe de guidage (44),
un élément de limitation (48),
un coupleur d'actionnement (60) relié au fil d'entraînement (22),
un dispositif de retenue (46) couplé à la pointe de guidage (44) et comprenant un loquet (50) à une extrémité distale, et
un élément de sollicitation (52) couplé à la pointe de guidage (44) et au dispositif de retenue (46) ; et
un calibreur (30, 96) couplé de manière amovible au coupleur de calibreur (28, 94) ;
dans laquelle (a), lorsque le mécanisme d'actionnement (18) exerce la force de traction sur le fil d'entraînement (22) pour le rétracter, le coupleur de calibreur (28) pivote autour du point de pivotement (62) dans une première direction de rotation (70) ; et
dans laquelle (b), lorsque le mécanisme d'actionnement (18) exerce une force de poussée sur le fil d'entraînement (22), le coupleur de calibreur (28) pivote autour du point de pivotement (62) dans une deuxième direction de rotation (68) opposée à la première direction de rotation (70).

2. Sonde de calibrage chirurgicale (10, 76) selon la revendication 1, dans laquelle le coupleur de calibreur (28, 94) comprend en outre une butée (58).

3. Sonde de calibrage chirurgicale (10, 76) selon la revendication 1 ou 2, dans laquelle le coupleur de calibreur (28, 94) comprend en outre un premier jeu intérieur (54).

4. Sonde de calibrage chirurgicale (10, 76) selon la revendication 3, dans laquelle le premier jeu intérieur (54) est situé entre le dispositif de retenue (46) et la pointe de guidage (44).

5. Sonde de calibrage chirurgicale (10, 76) selon l'une quelconque des revendications 1 à 4, dans laquelle le coupleur de calibreur (28, 94) comprend en outre un deuxième jeu intérieur (56).

6. Sonde de calibrage chirurgicale (10, 76) selon la revendication 5, dans laquelle le deuxième jeu intérieur (56) est situé entre le dispositif de retenue (46) et l'élément de limitation (48).

7. Sonde de calibrage chirurgicale (10, 76) selon l'une quelconque des revendications 1 à 6, dans laquelle la pointe de guidage (44) et l'élément de limitation (48) sont couplés par un pont à courbure interne.

8. Sonde de calibrage chirurgicale (10, 76) selon l'une quelconque des revendications 1 à 7, dans laquelle le coupleur de calibreur (28, 94) est formé d'une seule pièce.

9. Sonde de calibrage chirurgicale (10, 76) selon l'une quelconque des revendications 1 à 8, dans laquelle le calibreur (30, 96) comprend en outre un cylindre creux.

10. Sonde de calibrage chirurgicale (10, 76) selon l'une quelconque des revendications 1 à 9, dans laquelle le calibreur (30, 96) comprend en outre un élément de montage (32) couplé à une circonférence intérieure (38) du calibreur (30, 96).

11. Sonde de calibrage chirurgicale (10, 76) selon la revendication 10, dans laquelle l'élément de montage (32) comprend en outre une ouverture (36).

12. Sonde de calibrage chirurgicale (10, 76) selon l'une quelconque des revendications 1 à 11, dans laquelle le calibreur (30, 96) comprend en outre un élément d'accrochage de calibreur (34).

13. Sonde de calibrage chirurgicale (10, 76) selon l'une quelconque des revendications 1 à 12, dans laquelle le mécanisme d'actionnement est un bouton rotatif (18).

14. Sonde de calibrage chirurgicale (10, 76) selon l'une quelconque des revendications 1 à 13, dans laquelle la tige coudée (26, 92) est articulable.
